# EUROPEAN PATENT APPLICATION

(11) **EP 0 565 722 A1**
(43) Date of publication of application: **20.10.1993**
(21) Application number: 92901907.3
(22) Date of filing: 25.12.1991
(51) Int. Cl.: A61K 37/02, A61K 9/08, A61K 9/00

(54) **PHARMACEUTICAL PREPARATION FOR PERNASAL ADMINISTRATION**

(30) Priority: 25.12.1990 JP 405797/90
(71) Applicant: KYOWA HAKKO KOGYO CO., LTD., Chiyoda-ku, Tokyo 100 (JP)
(72) Inventor: HAYAKAWA, Eiji 495-15, Chabatake, Shizuoka 410-11 (JP); KATO, Yasuki 2-8-7, Senpukugaoka, Shizuoka 410-11 (JP); ITO, Kunio 174-13, Nameri, Sunto-gun Shizuoka 411 (JP); OKABE, Masami 30-5, Kamo, Shizuoka 411 (JP); ASANO, Makoto 3198-3-302, Ooka, Shizuoka 410 (JP)
(74) Representative: Casalonga, Axel
(86) International application number: JP9101757
(87) International publication number: WO9211022

(57) **Abstract**

A pharmaceutical preparation for the pernasal administration of a granulocyte colony-stimulating factor (G-CSF) to patients conveniently without being accompanied by any pain; and a method for increasing leukocytes and a treatment of infectious diseases by using the same. Although G-CSF has a molecular weight of as high as about 19,000, the preparation can be readily absorbed into the body by pernasal administration and hence is expected to be effective in treating leukopenia, in particular neutropenia.

## Description

### [Field of the Invention]

The present invention relates to a pharmaceutical composition for nasal administration which contains, as an effective component, a granulocyte colony-stimulating factor (hereinafter referred to as G-CSF), as well as to a method for increasing the number of leukocytes and a treatment for infectious diseases by using the same.

### [Background of the Invention]

G-CSF is known to be effective in the treatment for leukopenia caused by antitumor agents and other drugs, in the treatment for recovery of hematopoietic system after bone marrow transplantation, and in the treatment for various kinds of infectious diseases.

Heretofore, no methods have been known for administration of G-CSF in the clinical treatments, other than administration through injection. However, administration through injection must be made only by a doctor, etc. and is also painful for the patient who receives the administration. As continuous administration is also required in many cases for an effective application of G-CSF to patients, a simpler method of administration has been desired.

Recently, study on oral administration of G-CSF was reported (Hideaki Nomura, et al., Lecture Summaries of 110th Annual Meeting of the Pharmaceutical Society of Japan, Volume 4, p.84, 1990), but its pharmaceutical effects are exhibited only when used together with an absorption enhanser such as a surfactant, presenting the problem of side effects due to the surfactant.

Nasal administration of drugs having a relatively low molecular weight of up to 2000 has been reported, but drugs having a relatively higher molecular weight of 10,000 or higher have an extremely low nasal absorption rate (Delivery System for Peptide Drugs, Series A: Life Science, 125, 255-263), and are thus considered to be difficult for nasal administration. G-CSF has a molecular weight of about 19,000, and no reports have been found regarding its nasal administration.

An object of the present invention is to provide a method for easily administering G-CSF to patients without pain, and a composition for administration thereof.

### [Disclosure of the Invention]

As a result of intensive research into administration of G-CSF by methods other than injection, the present inventors have found that, although G-CSF has a molecular weight of as high as about 19,000, it is easily absorbed into the body by nasal administration, and simultaneously exhibits its pharmaceutical effect in increasing the number of leukocytes.

The present invention relates to a method for the nasal administration of G-CSF for hematopoietic recovery, and more particularly to a method for increasing the number of neutrophil or a method for promoting neutrophil functions, a method for the treatment for infectious diseases and a pharmaceutical composition used in these methods.

G-CSF used in the present invention includes all extracts from natural sources, sources of G-CSF obtained using recombinant DNA techniques, derivatives of intact G-CSF in which a portion of the amino acid sequence is substituted by other amino acids, or which has a deletion or addition, etc., so long as the peptide is included in the definition of a granulocyte colony stimulating factor.

These G-CSF substances may be used whether or not sugar chains are present thereon. The amino acid sequence of intact G-CSF is disclosed in the specification of U.S. Patent No. 4,883,127.

Specific examples of such derivatives which has been known and may be used in the present invention include a derivative in which the N-terminal amino acids Thr¹, Leu³, Gly⁴, Pro⁵ and Cys¹⁷ of the amino acid sequence of intact G-CSF are substituted by Ala¹, Thr³, Tyr⁴, Arg⁵ and Ser¹⁷, respectively [hereinafter referred to as (Ala¹, Thr³, Tyr⁴, Arg⁵, Ser¹⁷)G-CSF, and the following derivatives are referred to in the same manner], (Tyr¹, Ile³, Arg⁴, Ser⁵, Ser¹⁷)G-CSF, (Ala¹, Thr³, Tyr⁴, Arg⁵, Ser¹⁷)G-CSF, (Ile¹, Thr³, Arg⁴, Ser⁵, Ser¹⁷)G-CSF and (Arg⁴, Ser¹⁷)G-CSF, etc. (Japanese Published Unexamined Patent Application No. 267292/88); and (Ala¹)G-CSF, (Ser³⁶)G-CSF, (Ser⁴²)hpG-CSF, (Ser⁶⁴)hpG-CSF, (Ser⁷⁴)hpG-CSF, (Met⁻¹, Ser¹⁷)hpG-CSF, (Met⁻¹, Ser³⁶)hpG-CSF, (Met⁻¹, Ser⁴²)hpG-CSF, (Met⁻¹, Ser⁶⁴)hpG-CSF and (Met⁻¹, Ser⁷⁴)hpG-CSF (U.S. Patent No. 4,810,643), etc.

G-CSF is known to possess hematopoietic activity, and particularly to increase the number of neutrophils. There have been some reports regarding the pharmaceutical activity, toxicity, etc. of these G-CSF.

The acute toxicity of G-CSF in mice, rats and monkeys is 3 mg/kg or greater, and the dose for nasal administration is 0.5-5 mg per day for adults.

The pharmaceutical activity of (Ala¹, Thr³, Tyr⁴, Arg⁵, Ser¹⁷)G-CSF (hereinafter referred to as ND28), etc. are described in detail in Biochem. Biophys. Res. Commun., 159, 103, 1989, Blood, 75, 1788, 1990, Pharmacology and Therapy, Vol. 19, No. 6, etc., and its toxicity is described in Applied Pharmacology, Vol. 41, No. 4, etc.

ND28 is known to be expected for clinical effects, including an infection-preventing effect against Gram-negative and Gram-positive bacteria, and both an infection-preventing effect and an infection-curing effect against fungi (C.alubicans) [Kiso to Rinsho (Experiments and Clinics), Vol. 25, No. 13].

Nasal administration is a method of administration by which a drug is absorbed through nasal mucosa, and a phamaceutical composition of the present invention also may be used in the form of a liquid or powder suitable for nasal administration.

In the case of a liquid preparation, it is applied to the nasal mucosa by dropping, spraying or swabbing, etc., while in the case of a powder preparation, it is used by a method such as spraying.

The composition of the present invention usually comprises G-CSF and a pharmaceutically acceptable diluent, adjuvant, carrier, etc. The liquid preparation may contain additives such as pH adjusters, antiseptics, soothing agents, toxicity adjusters, antioxidants, and stabilizers, etc. The powder preparation may contain excipients such as sugars, starches, celluloses, pH adjusters, antiseptics, soothing agents, antioxidants, toxicity adjusters, and stabilizers.

The pharmaceutical composition of the present invention may be produced by conventional methods used in the production of composition for nasal administration. For example, a liquid preparation may be obtained by dissolving G-CSF in distilled water or in an appropriate buffer solution such as a phosphate buffer solution, acetate buffer solution, borate buffer solution, citrate buffer solution, and lactate buffer solution, and subjecting the solution to aseptic filtration with a 0.2 µm pore membrane filter. The amount of G-CSF contained in the liquid preparation is 0.001-100 mg/ml, and preferably 0.01-10 mg/ml. A powder preparation may be obtained, for example, by dissolving G-CSF and an excipient in a suitable buffer solution, subjecting the solution to aseptic filtration, dispensing it into containers for administration and subjecting the solutions to freeze drying, or by dissolving G-CSF and a starch, cellulose, sugar, etc. in a buffer solution such as a phosphate, acetate, borate, citrate and lactate buffer solution, subjecting the solution to aseptic filtration and freeze-drying and pulverizing the product thereof. The amount of G-CSF in the powder preparation is 0.0001-100% by weight, and preferably 0.001-10% by weight. The powder preparation may be used as a liquid preparation by dissolving at the time of use, if necessary.

The composition of the present invention is quickly absorbed after its administration, and is capable of effectively increasing the leukocytes in blood. In addition, it can cure leucopenia, a side effect caused by antitumor agents and other drugs, and furthermore said preparation can be administered to patients without pain even when G-CSF must be administered over a long period of time, such as in the case of diseases such as congenital neutropenia, anaplastic anemia, and various infectious diseases.

### [Brief Description of the Drawings]

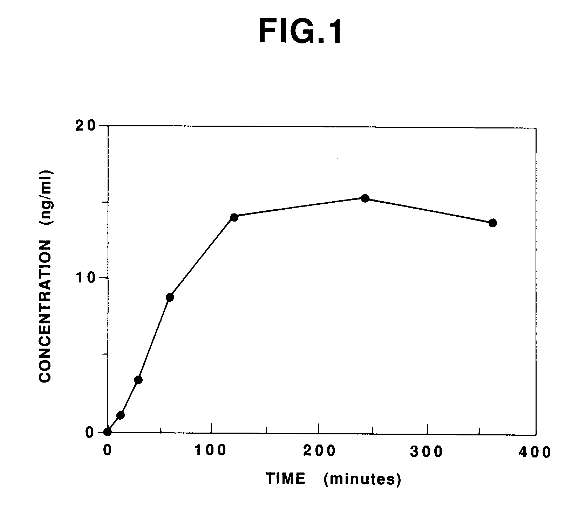

Fig. 1 shows the change in concentration of ND28 in the plasma with the elapse of time after nasal administration of the liquid preparation obtained in Example 1 to rats, according to the method in Experiment 1.

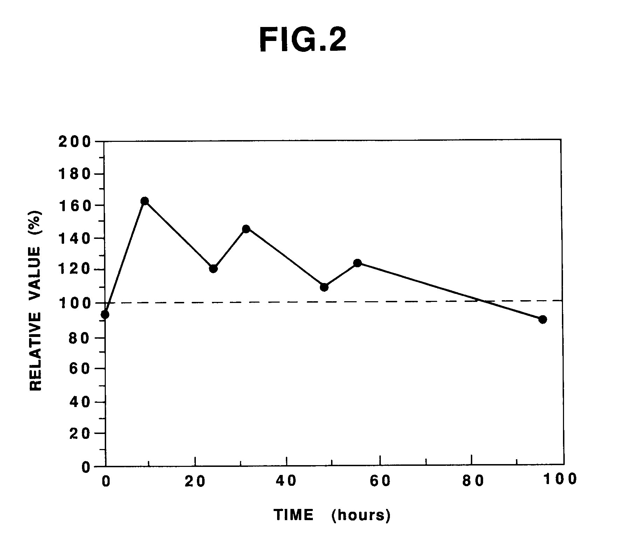

Fig. 2 shows the change in the number of leukocytes with the elapse of time after nasal administration of the liquid preparation obtained in Example 1 to mice, according to the method in Experiment 2. The number of leukocytes in the case of administration of the control preparation for nasal administration is defined as 100%.

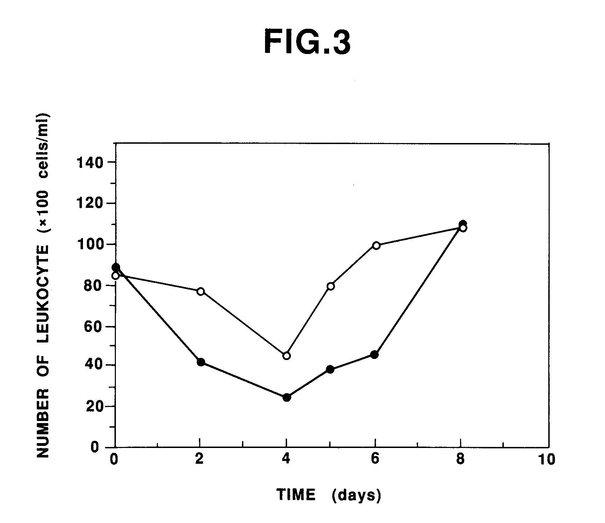

Fig. 3 shows the change in the number of leukocytes with the elapse of time after nasal administration of ND28 to mice with cyclophosphamide-induced leucopenia, according to the method in Experiment 3. The open circles indicate the preparation for nasal administration obtained in Example 2, and the closed circles indicate the control preparation for nasal administration.

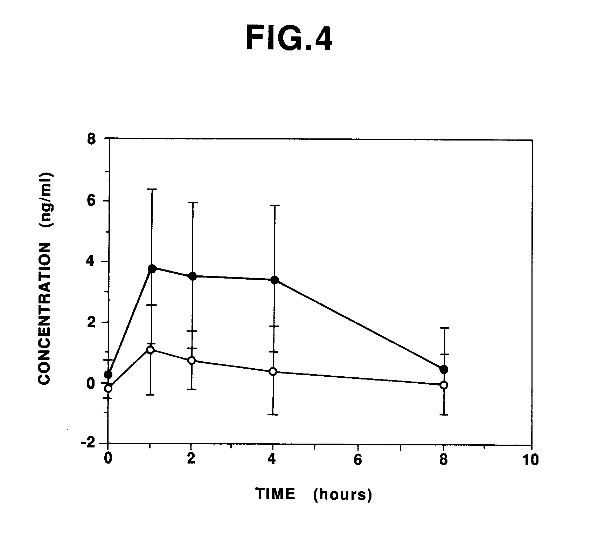

Fig. 4 shows the change in the concentration of G-CSF in the plasma with the elapse of time after nasal administration of the liquid preparations obtained in Examples 8 and 9 to rats, according to the method in Experiment 4. The open circles indicate the preparation obtained in Example 9, and the closed circles indicate the liquid preparation obtained in Example 8.

### [Specific Embodiment of the Invention]

### Example 1

(Ala¹, Thr³, Tyr⁴, Arg⁵, Ser¹⁷)G-CSF (ND28) was dissolved in a 20 mM phosphate buffer solution adjusted to pH 7.2 in the proportion of 1 mg to 1 ml. The solution was subjected to aseptic filtration with a 0.2 µm disposable membrane filter (product of Germane Science Co.), and the filtrate was dispensed into plastic containers in portions of 1 ml each to obtain liquid preparations.

### Example 2

ND28 was dissolved in distilled water in the proportion of 1 mg to 1 ml, and the solution was subjected to aseptic filtration with a 0.2 µm disposable membrane filter. The filtrate was dispensed into glass vials in portions of 2 ml each, and then freeze-dried to obtain freeze-dried preparations.

### Example 3

ND28 and lactose were dissolved in a phosphate buffer solution of pH of approximately 7 in the proportions of 0.5 mg and 100 mg, respectively, to 2 ml. The solution was treated in the same manner as in Example 2 to obtain freeze-dried preparations.

### Example 4

ND28 and lactose were dissolved in a phosphate buffer solution adjusted to pH 4.5 in the proportions of 0.5 mg and 100 mg, respectively, to 2 ml. The solution was subjected to aseptic filtration with a 0.2 µm disposable membrane filter and dispensed into plastic containers in portions of 0.2 ml each and then freeze-dried to obtain freeze-dried preparations.

### Example 5

Intact human G-CSF and lactose were dissolved in a phosphate buffer solution adjusted to pH 7 in the proportions of 0.1 mg and 100 mg, respectively, to 2 ml. The solution was treated in the same manner as in Example 2 to obtain freeze-dried preparations.

### Example 6

ND28 and mannitol were dissolved in distilled water in the proportions of 50 µg and 40 mg, respectively, to 1 ml. The solution was subjected to aseptic filtration with a 0.2 µm disposable membrane filter and dispensed into glass vials in portions of 1 ml each and then freeze-dried to obtain freeze-dried preparations.

### Example 7

ND28 and sodium chloride were dissolved in a phosphate buffer solution adjusted to pH 7 in the proportions of 1 mg and 0.9 mg, respectively, to 1 ml. The solution was freeze-dried and pulverized with a mortar to obtain a powder preparation.

### Example 8

ND28 was dissolved in a 20 mM phosphate buffer solution adjusted to pH 7.2 in the proportion of 40 µg to 1 ml. The solution was subjected to aseptic filtration with a 0.2 µm disposable membrane filter (manufactured by Germane Science Co.), and was dispensed into plastic containers in portions of 1 ml each to obtain liquid preparations.

### Example 9

Intact human G-CSF was dissolved in a 20 mM phosphate buffer solution adjusted to pH 7.2 in the proportion of 40 µg to 1 ml. The solution was subjected to aseptic filtration with a 0.2 µm disposable membrane filter (manufactured by Germane Science Co.), and was dispensed into plastic containers in portions of 1 ml each to obtain liquid preparations.

The change in concentration in blood and increase in the number of leukocyte after nasal administration of the preparations of the present invention are shown by referring to the Experiments.

The concentration in blood of G-CSF which had been absorbed into the body by nasal administration was determined by the NFS60-MTT method described below or by a method of determining the number of leukocytes in blood with an automatic cell counter.

### NFS60-MTT Method

NFS60 cells taken from mice are poured into each well of a 96-well microplate at a concentration of 4 x 10⁵ cells/ml in an amount of 50 µl per well. Then a standard solution and test solution which have been previously diluted to have theoretical G-CSF concentrations of 5 ng/ml are subjected to two-fold serial dilution in each of the wells on the plate. The cells are incubated in a 5% CO₂ incubator at 37°C for about 40 hours. Next, MTT [3-(4,5-dimethylimidazol-2-yl)-2,5-diphenyltetrazolium bromide] is dissolved in phosphate-buffered physiological saline to a concentration of 2 mg/ml, and the MTT solution is added in an amount of 10 µl to each of the wells of the microplate. The cells are further incubated in a 5% CO₂ incubator at 37°C for about 5 hours. Then, 125 µl of dimethylsulfoxide is added to each of the wells, and the resulting formazan is dissolved. After the dissolution, the absorbance of each of the plate wells at 550 nm is measured by a microplate photometer. The absorbance are compared with that of the standard solution to determine the G-CSF concentration of the test solution.

### Experiment 1

Cannulas were inserted into the jugular arteries of male Wistar rats under urethane anesthesia. Using a micropipette, 50 µl of the liquid preparation obtained in Example 1 was dropped into the nasal cavities of the rats. The blood was collected at appropriate intervals through the jugular arteries, heparinized and centrifuged to obtain plasma. The obtained plasma was diluted with RPMI1640 culture medium containing 10% fetal calf serum, and the G-CSF concentration in the diluted solution was determined by NFS60-MTT method.

The results are shown in Fig. 1. At 15 minutes after nasal administration of 50 µg of ND28 per rat, approximately 1 ng/ml of ND28 was measured in the plasma, and ND28 concentration increased with the elapse of time to reach approximately 14 ng/ml at 2 hours after administration. The concentration of ND28 in the plasma remained the constant value until 6 hours after administration.

From the above results, it is clear that G-CSF is easily absorbed by nasal administration, and appears at a high concentration in blood.

### Experiment 2

Using a microsyringe, 50 µl of the liquid preparation obtained in Example 1 was dropped into the nasal cavities of male BALB/c mice. As a control, 50 µl of a liquid preparation for nasal administration, which was obtained by subjecting physiological saline to aseptic filtration with a 0.2 µm disposable membrane filter, was also administered to mice. Blood was taken at appropriate intervals from the eyeground vein, and the number of leukocytes therein was measured with an automatic cell counter (CC-180A, manufactured by Toa Iryo Denshi Co.).

The results are shown in Table 1 and in Fig. 2. From the 9th hour to the 55th hour after nasal administration of 50 µg of ND28 per mouse, a clear increase in the number of leukocytes was observed in comparison to the control group.

**Table 1**

| | Time after administration (hours) | | | | | | |
|---|---|---|---|---|---|---|---|
| | 0 | 9 | 24 | 31 | 48 | 55 | 96 |
| Example 1 | 102.9 | 100.6 | 142.2 | 116.9 | 127.2 | 88.9 | 60.4 |
| Control | 110.6 | 62.0 | 118.4 | 80.8 | 117.0 | 72.6 | 68.6 |
| Units: x 10² cells/ml | | | | | | | |

### Experiment 3

Male BALB/c mice were intraperitoneally administered 100 mg/kg of cyclophosphamide. From the day after the administration, the preparation obtained in Example 2 was dissolved in 1 ml of distilled water for injection, and the liquid preparation was dropped into the nasal cavities of the mice using a micropipette, 50 µl at a time once a day for 4 days. As a control, a liquid preparation for nasal administration obtained by subjecting distilled water for injection to aseptic filtration with a 0.2 µm disposable membrane filter was administered in the same manner. Blood was taken from the eyeground veins of the mice on the 2nd, 4th, 5th, 6th and 8th days after administration of the cyclophosphamide, and the number of leukocytes was determined by an automatic cell counter.

The results are shown in Fig. 3. The group to which 100 µg of ND28 was administered per mouse recovered more quickly from the cyclophosphamide-induced leucopenia in comparison with the control group. From the above results, it is clear that G-CSF preparation for nasal administration is effective for the treatment of leucopenia, a side effect caused by antitumor agents.

### Experiment 4

Cannulas were inserted into the jugular arteries of male Wistar rats under urethane anesthesia. The liquid preparations obtained in Example 8 and Example 9 were diluted with distilled water to a concentration of 2 µg per 50 µl in terms of protein, and using a micropipette, 50 µl each was dropped into the nasal cavities of the rats. Blood was taken at appropriate intervals through the jugular arteries, and was then heparinized and centrifuged to obtain the plasma. The obtained plasma was diluted with RPMI1640 culture medium containing 10% fetal calf serum, and the G-CSF concentration in the diluted solution was measured by NFS60-MTT method.

The results are shown in Fig. 4. The preparation for nasal administration containing ND28 exhibited a higher G-CSF concentration in blood than the one containing intact G-CSF.

### [Industrial Applicability]

According to the present invention, there is provided G-CSF composition for nasal administration, which is useful for the treatment of leucopenia caused by antitumor agents and other drugs, for the treatment of recovery of hematopoietic system after bone marrow transplantation and for the treatment of various kinds of infectious diseases, etc. and which is easy to administer.

## Claims

1. A pharmaceutical composition for nasal administration which contains, as an effective component, a granulocyte colony stimulating factor.

2. A pharmaceutical composition according to Claim 1 wherein the granulocyte colony stimulating factor is (Ala¹, Thr³, Tyr⁴, Arg⁵, Ser¹⁷)G-CSF.

3. A pharmaceutical composition according to Claim 1 which is in the form of an aqueous solution.

4. A pharmaceutical composition according to Claim 1 which is in the form of a powder.

5. A method for the treatment of leucopenia wherein a pharmaceutical composition comprising an effective amount of a granulocyte colony stimulating factor and a pharmaceutically acceptable diluent, adjuvant or carrier is administered through nasal cavity.

6. A method according to Claim 5 wherein the granulocyte colony stimulating factor is (Ala¹, Thr³, Tyr⁴, Arg⁵, Ser¹⁷)G-CSF.
